# EUROPÄISCHE PATENTSCHRIFT

(11) **EP 4 384 111 B1**
(45) Veröffentlichungstag und Bekanntmachung des Hinweises auf die Patenterteilung: **25.12.2024**
(21) Anmeldenummer: 23758617.7
(22) Anmeldetag: 18.08.2023
(51) Int. Cl.: A61B 50/30, B25J 15/00, A61B 50/00

(54) **HANDHABUNGSWERKZEUG FÜR MEDIZINISCHE GERÄTSCHAFTEN**
HANDLING TOOL FOR MEDICAL EQUIPMENT
OUTIL DE MANIPULATION POUR ÉQUIPEMENT MÉDICAL

(30) Priorität: 22.08.2022 DE 102022121160
(43) Veröffentlichungstag der Anmeldung: 19.06.2024
(73) Patentinhaber: Aesculap AG, 78532 Tuttlingen (DE)
(72) Erfinder: JANSEN-TROY, Arne, 78333 Stockach (DE); MAAS, Allan, 78467 Konstanz (DE); KIESSLING, Daniel, 78052 Villingen-Schwenningen (DE); HENKE, Matthias, 78048 Villingen-Schwenningen (DE)
(74) Vertreter: Winter, Brandl - Partnerschaft mbB
(86) Internationale Anmeldenummer: PCT/EP2023/072804
(87) Internationale Veröffentlichungsnummer: WO 2024/041997

(56) Entgegenhaltungen:
- WO-A1-2021/018649
- GB-A- 2 591 071
- US-A1- 2016 151 123
- US-A1- 2020 016 770

## Beschreibung

### Technisches Gebiet

Die vorliegende Offenbarung betrifft ein Handhabungswerkzeug zur Handhabung einer medizinischen Gerätschaft/eines medizinischen Produkts/eines medizinischen Objekts, insbesondere einem medizinischen Behälter, und ein System aus dem Handhabungswerkzeug und dem medizinischen Behälter.

### Hintergrund der vorliegenden Offenbarung

Medizinische Behälter wie beispielsweise Sterilgutcontainer, Siebkörbe mit Weichverpackungen und dergleichen werden bereits automatisiert gehandhabt. Eine derartige Handhabung erfolgt mittels paralleler oder serieller Roboter mit entsprechenden Manipulatoren, Flurförderfahrzeugen, AGV (Automated Guided Vehicles), Förderbahnen und -bändern, Aufzugsystemen und weiteren in der Logistik weit verbreiteten Fördertechnologien.

In vielen Fällen kommt in hochautomatisierten AEMPs (Aufbereitungseinheit für Medizinprodukte) eine Tragevorrichtung in Form eines Tablets zum Einsatz, auf welchem sich das zu bewegende Objekt oder Produkt befindet. Dies ermöglicht ein sicheres Handhaben unabhängig von einer Geometrie des Objekts oder Produkts. Nachteilig ist jedoch, dass die Tablets einen zusätzlichen Kosten-, Reinigungs- und Logistikaufwand darstellen.

Werden Objekte in Form von Sterilgutcontainern direkt gehandhabt, so erfolgt dies bisher mit einem schaufelartig ausgeprägten Werkzeug, welches unter den Boden des Objekts fährt. Nachteilig ist hier, dass ein zusätzlicher Mechanismus zum Anheben des Objekts eingesetzt werden muss, um das schaufelartig ausgeprägte Werkzeug unter dem Objekt zu platzieren. Ein Greifen von einer Tischplatte und/oder aus einem Regal und/oder einem Containertransportwagen ist mit diesem Werkzeug nicht möglich, da das Objekt durch das schaufelartig ausgeprägte Werkzeug verschoben werden kann, bevor das Objekt auf der Schaufelfläche aufliegt. Weiterhin ist bei dem schaufelartig ausgeprägten Werkzeug eine maximale Verfahr-/Handhabungsgeschwindigkeit, bei welcher ein Verrutschen des Objekts ausgeschlossen werden kann, limitiert.

In allgemeinen Logistikanwendungen haben sich für derartige Anwendungen Gabelstapler bzw. Hubvorrichtungen mit flächigen Gabelzinken durchgesetzt, welche parallelbeabstandet sind und so eine theoretische Auflageebene aufspannen. Nachteilig an derartigen flächigen Gabelzinken ist jedoch, dass in einem Boden des zu transportierenden Objektes Huböffnungen zur Aufnahme der Gabelzinken ausgebildet sein müssen, wodurch ein nutzbares Innenvolumen des Objekts reduziert ist. Alternativ hierzu müsste der anzuhebende Gegenstand (Objekt) auf einer Palette abgestellt sein, die für ein Unterfahren der Gabelzinken profiliert sein müsste. Weiterhin ist durch die lediglich auf Kraftschluss basierende Fixierung des Objekts auf den Gabelzinken die maximale Verfahr-/Handhabungsgeschwindigkeit, bei welcher das Verrutschen des Objekts ausgeschlossen werden kann, limitiert, es sei denn, das Objekt wird zusätzlich gegen Verrutschen gesichert, was aber wieder aufwändig wäre.

Die US 2020 / 016 770 A1 offenbart eine Handhabungsvorrichtung für Behälter mit Gabelzinken, wobei an einer Oberseite der Gabelzinken Aufnahmevorsprünge ausgebildet sind. Auch aus der WO 2021 / 018 649 A1, der GB 2 591 071 A und der US 2016 / 151 123 A1 sind entsprechende Handhabungsvorrichtungen für Behälter mit Gabelzinken und Aufnahmegeometrien bekannt.

### Kurzbeschreibung der Offenbarung

Aufgabe der vorliegenden Offenbarung ist demnach, die Nachteile aus dem Stand der Technik zu beheben oder zumindest zu reduzieren. Konkret ist es Aufgabe der vorliegenden Offenbarung, ein Handhabungswerkzeug für zu handhabende Objekte, insbesondere medizinische Gerätschaften, bereitzustellen, welches ein Greifen bzw. Aufnehmen des Objekts von einem beliebigen Untergrund bzw. unabhängig von einer Positionierung des Objekts sowie eine hohe Handhabungsgeschwindigkeit des Objekts, insbesondere bei einer automatisierten Handhabung, ermöglicht.

Der Begriff "Greifen" ist an dieser Stelle zunächst ganz allgemein zu verstehen und beschränkt sich nicht nur auf einen Zangen-förmigen Griff mit welchem ein Objekt zwischen zwei Branchen eingeklemmt wird sondern jedwede Aufnahmeart also auch das einfache (lose) Untergreifen eines Objekts, bei welchem lediglich ein Kraft in Hubrichtung auf das Objekt aufgebracht wird oder das Eingreifen in entsprechende Aufnahmen am Objekt, bei welchem zwar nur eine Hubkraft auf das Objekt aufgebracht wird aber gleichzeitig ein seitlichen Ver-/Abrutschen des Objekts durch entsprechende Führungen unterbunden werden kann.

Die vorstehende Aufgabe wird daher gelöst durch ein vorzugsweise medizinisches Handhabungswerkzeug nach Anspruch 1 sowie durch ein System nach Anspruch 11. Vorteilhafte Weiterbildungen sind in den Unteransprüchen offenbart, deren Inhalte ggf. getrennt sowie unabhängig beansprucht werden können.

Konkret wird die Aufgabe gelöst durch das vorzugsweise medizinische Handhabungswerkzeug, das zum Greifen und/oder Handhaben einer medizinischen Gerätschaft/eines medizinischen Objekts/Produkts/Gegenstands, vorzugsweise eines medizinischen Behälters, weiter vorzugsweise Sterilisationsbehälters, vorgesehen und ausgebildet ist, mit einem Gabelträger und zwei sich in einer Längsrichtung rechtwinklig weg vom Gabelträger erstreckende, parallelbeabstandete Gabelzinken, von denen jeder eine Behälterauflage-/ erfassungsseite hat, an der eine Anzahl längsbeabstandeter Aufnahmevorsprünge ausgebildet oder angeordnet sind, die sich in einer (positiven) Höhenrichtung (also nach oben) zur Behälterauflage-/ erfassungsseite erstrecken.

In anderen Worten beinhaltet das Handhabungswerkzeug den Gabelträger, der sich im Wesentlichen symmetrisch, ausgehend von einer Mittelposition, in einer positiven und in einer negativen Breitenrichtung erstreckt. An jeweiligen Endabschnitten des Gabelträgers in Breitenrichtung ist jeweils ein Gabelzinken ausgebildet, der sich in der positiven Längsrichtung weg von dem Gabelträger erstreckt. In nochmals anderen Worten bilden der Gabelträger und die beiden Gabelzinken im Wesentlichen eine U-Form, wobei die zu handhabende medizinische Gerätschaft im Wesentlichen in einem Aufnahmeraum, welcher zwischen den Gabelzinken ausgebildet ist, aufgenommen wird.

Die Gabelzinken beinhalten die Aufnahmevorsprünge, welche sich in der Höhenrichtung weg von den Gabelzinken erstrecken. Unter der Höhenrichtung ist eine Richtung zu verstehen, die im Wesentlichen normal zu einer von der Längsrichtung und der Breitenrichtung aufgespannten Ebene orientiert ist. Die Aufnahmevorsprünge sind durch Aussparungen voneinander getrennt. Vorzugsweise sind zumindest zwei Aufnahmevorsprünge pro Gabelzinken ausgebildet. Vorzugsweise ist in einem globalen Koordinatensystem eine Ausdehnung des Gabelträgers in der Höhenrichtung größer als in der Längsrichtung. Weiterhin vorzugsweise ist in dem globalen Koordinatensystem eine Ausdehnung des Gabelzinkens in der Höhenrichtung größer als in der Breitenrichtung.

Kern der Offenbarung ist demzufolge das Handhabungswerkzeug, bestehend aus dem Gabelträger und den zwei Gabelzinken, wobei die Gabelzinken jeweils mit den (nach oben sich erstreckenden) Aufnahmevorsprüngen ausgebildet sind.

Durch die Ausbildung der Gabelzinken mit den Aufnahmevorsprüngen kann eine formschlüssige Aufnahme/Verrastung der zu handhabenden medizinischen Gerätschaft auf den Gabelzinken erzielt werden, wodurch eine Erhöhung einer Handhabungsgeschwindigkeit bei gleichzeitiger Erhöhung einer Handhabungssicherheit erzielt werden kann.

In einem ersten Aspekt können sich die Aufnahmevorsprünge der Gabelzinken derart verjüngt, insbesondere keilförmig, ausgebildet sein, dass eine Höhenerstreckung der Aufnahmevorsprünge in Höhenrichtung an zueinander zugewandten Seiten der Gabelzinken größer ist als eine Höhenerstreckung der Aufnahmevorsprünge in Höhenrichtung an voneinander abgewandten Seiten der Gabelzinken.

Anders ausgedrückt können die Aufnahmevorsprünge der Gabelzinken sich in Höhenrichtung zueinander hin, insbesondere bezogen auf eine Ausdehnung in einer Breitenrichtung, welche einer Erstreckungsrichtung des Gabelträgers entspricht, verjüngen.

In nochmals anderen Worten können die Aufnahmevorsprünge eine Rampengeometrie aufweisen, deren Höhe in Richtung zu dem zwischen den Gabelzinken ausgebildeten Aufnahmeraum hin ansteigt. Die Rampengeometrie kann bevorzugt eine im Wesentlichen lineare Kontur aufweisen. Alternativ kann die Rampengeometrie eine (teil-) parabelförmige Kontur aufweisen.

Durch eine derartige Ausbildung der Aufnahmevorsprünge der Gabelzinken kann ein Einrastvorgang der Gabelzinken in die von dem Handhabungswerkzeug zu handhabende medizinische Gerätschaft verbessert werden. Weiterhin können durch die derartige Ausbildung Fertigungstoleranzen, Positionierungstoleranzen und/oder ein Verzug der zu handhabenden medizinischen Gerätschaft ausgeglichen werden. Zusätzlich kann durch eine derartige Geometrie eine Selbsthemmung der (Rast-) Verbindung zwischen den Aufnahmevorsprüngen und der zu handhabenden medizinischen Gerätschaft erzielt werden.

In einem weiteren Aspekt beinhalten die Gabelzinken eine von der Behälterauflage-/ erfassungsseite abgewandte Unterseite, an welcher Manipulationsvorsprünge ausgebildet oder angeordnet sind, die sich in einer Tiefenrichtung / negativen Höhenrichtung zur Unterseite erstrecken und welche vorzugsweise derart verjüngt, insbesondere keilförmig, ausgebildet sind, dass eine Höhenerstreckung der Manipulationsvorsprünge in Höhenrichtung an den zueinander zugewandten Seiten der Gabelzinken kleiner ist als eine Höhenerstreckung der Manipulationsvorsprünge in Höhenrichtung an den voneinander abgewandten Seiten der Gabelzinken .

Anders ausgedrückt beinhalten die Gabelzinken die von der Behälterauflage-/ erfassungsseite abgewandte Unterseite, an welcher die Manipulationsvorsprünge ausgebildet oder angeordnet sind, die sich in einer Tiefenrichtung zur Unterseite erstrecken und welche sich vorzugsweise in einer negativen Höhenrichtung voneinander weg, insbesondere bezogen auf eine Ausdehnung in der Breitenrichtung, verjüngen.

Anders ausgedrückt können die Gabelzinken die Manipulationsvorsprünge beinhalten, die sich in negativer Höhenrichtung weg von den Gabelzinken erstrecken. Die Manipulationsvorsprünge können vorzugsweise eine Rampengeometrie aufweisen, deren Erstreckung in negativer Höhenrichtung in Richtung zu dem zwischen den Gabelzinken ausgebildeten Aufnahmeraum hin abfällt. Die Rampengeometrie kann bevorzugt eine im Wesentlichen lineare Kontur aufweisen. Alternativ kann die Rampengeometrie eine (teil-) parabelförmige Kontur aufweisen.

Die Manipulationsvorsprünge ermöglichen ein Manipulieren einer Position bzw. ein Schieben oder Ziehen der zu handhabenden medizinischen Gerätschaft, wenn sich die medizinische Gerätschaft nicht zwischen den Gabelzinken befindet.

In einem weiteren Aspekt können Manipulationsvorsprünge rotationssymmetrisch, insbesondere um 180 ° rotationssymmetrisch, zu den Aufnahmevorsprüngen, bezogen auf eine sich in Längsrichtung der Gabelzinken erstreckenden Mittelachse der Gabelzinken, ausgebildet sein.

Anders ausgedrückt kann die Kontur der Manipulationsvorsprünge der Kontur der Aufnahmevorsprünge in einem um 180° gedrehten Zustand entsprechen.

Durch eine derartige Gestaltung der Manipulationsvorsprünge können die Manipulationsvorsprünge auf eine Art entsprechend, der der Aufnahmevorsprünge in der zu handhabenden medizinischen Gerätschaft verhaken/verrasten, um die medizinische Gerätschaft zu schieben oder zu ziehen. Konkret ermöglichen derartige Manipulationsvorsprünge das Verhaken/Verrasten derart, dass nur einer der Gabelzinken mit den zugehörigen Manipulationsvorsprüngen im Eingriff ist, wenn das Handhabungswerkzeug um 180° zu einer Ursprungsposition gedreht ist.

In einem weiteren Aspekt kann das Handhabungswerkzeug, vorzugsweise in / an dem Gabelträger, eine Sensoraufnahme beinhalten.

In anderen Worten kann an dem Handhabungswerkzeug eine Aussparung oder dergleichen ausgebildet sein, welche vorgesehen ausgebildet ist, einen Sensor aufzunehmen. Bei dem Sensor kann es sich um einen optischen und/oder funkbasierten und/oder ultraschallbasierten Sensor handeln, welcher geeignet ist, die zu handhabenden Objekte, insbesondere medizinische Gerätschaften, beispielsweise anhand eines Tags / einer Markierung, zu erkennen.

Alternativ oder zusätzlich kann das Handhabungswerkzeug die Sensoraufnahme an zumindest einem Gabelzinken beinhalten.

Durch eine derartige Ausbildung des Handhabungswerkzeugs ist eine Unterbringung des obigen Sensors in/an dem Handhabungswerkzeug ermöglicht. Ein derartiger Sensor unterstützt bei einer Erfassung einer insbesondere relativen Position der medizinischen Gerätschaft in Bezug auf das Handhabungswerkzeug, wodurch ein automatisiertes und computergestützt definiertes Verfahren des Handhabungswerkzeugs ermöglicht ist.

In einem weiteren Aspekt kann ein Abstand zwischen den zwei Gabelzinken, vorzugsweise stufenlos, veränderlich sein.

Anders ausgedrückt können die Gabelzinken relativ zu dem Gabelträger in der Breitenrichtung, also in der Erstreckungsrichtung des Gabelträgers, beweglich sein. Bevorzugt können die Gabelzinken mittels zumindest einer Gewindestande oder zumindest einem Hydraulikzylinder oder zumindest einem Pneumatikzylinder beweglich sein.

Durch eine derartige Ausbildung des Handhabungswerkzeugs kann das Handhabungswerkzeug flexibel auf eine Vielzahl von medizinischen Gerätschaften angepasst werden, wodurch ein Einsatzspektrum des Handhabungswerkzeugs erheblich vergrößert wird.

In einem weiteren Aspekt kann zumindest einer der Gabelzinken an einem von dem Gabelträger abgewandten Endabschnitt ein Rastelement/eine Rastgeometrie, vorzugsweise einen Haken beinhalten.

In anderen Worten ist das Handhabungswerkzeug an zumindest einem der Gabelzinken, vorteilhafter Weise jedoch an beiden Gabelzinken, mit dem Rastelement ausgebildet, welches ein formschlüssiges Eingreifen in ein vorzugsweise zylinderförmiges Element ermöglicht. Konkret ermöglicht das Rastelement das formschlüssige Eingreifen in ein insbesondere zylinderförmiges Objekt, welches idealerweise parallel zu dem Gabelträger ausgerichtet ist. Bei dem Objekt kann es sich beispielsweise um einen Griff und/oder ein Interface und/oder eine Öse eines (medizinischen) Siebkorbs, eines (medizinischen) Drahtgestells, einer Box oder Ähnlichem handeln.

In einem weiteren Aspekt kann zumindest einer der Gabelzinken mit zumindest einem Klemmelement, vorzugsweise einem pneumatischen Kissen, ausgebildet sein.

In anderen Worten kann an zumindest einem der Gabelzinken, vorzugsweise jedoch an beiden Gabelzinken, das Klemmelement in Richtung zu dem zwischen den Gabelzinken ausgebildeten Aufnahmeraum hin ausgebildet sein, welches vorgesehen ausgebildet ist einen Kraftschluss zu der handzuhabenden medizinischen Gerätschaft herzustellen. Vorzugsweise kann das Klemmelement als ein pneumatisches oder hydraulisches Kissen ausgebildet sein, welches sich bei einer Druckbeaufschlagung ausdehnt und eine Klemmkraft auf die zu greifenden Produkte ausübt.

Durch eine Ausbildung der Gabelzinken mit dem zumindest einen Klemmelement kann ein Verrutschen der zu greifenden Produkte, vorzugsweise in Form von medizinischen Gerätschaften, unterbunden/verhindert werden. Dies ist besonders bei schweren zu greifenden Produkten, wie beispielsweise schwer beladenen Sterilgutcontainern, vorteilhaft, um bei einer Richtungsänderung des Handhabungswerkzeugs die zu greifenden Produkte sicher in/an dem Handhabungswerkzeug zu fixieren.

In einem weiteren Aspekt können die Gabelzinken eine Fachwerkstruktur (Leichtbaustruktur) aufweisen.

In anderen Worten können die Gabelzinken und/oder der Gabelträger mit einer belastungsgerechten Geometrie mit gewichtsreduzierenden Ausnehmungen ausgebildet sein, wobei unbelastete Bereiche ohne Material oder mit reduzierter Wandstärke ausgebildet sein können.

Durch eine derartige Ausbildung der Gabelzinken und/oder des Gabelträgers kann ein Gewicht des Handhabungswerkzeugs reduziert werden. Durch das geringere Gewicht bzw. eine daraus resultierende geringere Trägheit des Handhabungswerkzeugs kann die Handhabungsgeschwindigkeit erhöht werden.

In einem weiteren Aspekt kann der Gabelträger an einem von den Gabelzinken abgewandten Abschnitt ein Interface eines robotischen Systems beinhalten.

Anders ausgedrückt kann das Handhabungswerkzeug über den Gabelträger mittels des Interfaces, welches beispielsweise in Form eines Flansches ausgebildet ist, mit dem robotischen System verbunden/verbindbar sein. Das Interface kann so an dem Gabelträger ausgebildet sein, dass es in der Breitenrichtung mittig zwischen den Gabelzinken angeordnet ist.

In einem weiteren Aspekt kann das Interface ein drehbares/rotierbares Interface sein, sodass das Handhabungswerkzeug um eine Mittelachse des Interfaces, welche parallel zu der Längsrichtung orientiert ist, rotiert werden kann.

Das Handhabungswerkzeug kann weiterhin aus einem faser- oder glaskugelverstärkten Kunststoff ausgebildet sein. Alternativ kann das Handhabungswerkzeug aus einem metallischen Werkstoff ausgebildet sein.

In einem weiteren Aspekt kann das Handhabungswerkzeug spanend, im Schichtaufbau (Verbundkunststoff), im Spritzgussverfahren oder mittels Rapid-Prototyping (Additive Fertigung) ausgebildet sein.

In einem weiteren Aspekt können die Gabelzinken an einem von dem Gabelträger abgewandten Endabschnitt eine Fase beinhalten. Eine derartige Fase erleichtert einen Einschub des Gabelzinken zwischen eng aneinander positionierten zu greifenden Produkten und/ oder verhindert ein Verhaken der Gabelzinken. Weiterhin kann durch die Fase Material und damit Gewicht eingespart werden, was eine mechanische Belastung reduziert.

In einem weiteren Aspekt können die Gabelzinken an einem an den zwischen den Gabelzinken ausgebildeten Aufnahmeraum hin angrenzenden Abschnitt jeweils einen Anschlag, vorzugsweise in Form eines Radius, beinhalten.

In einem weiteren Aspekt können die Aufnahmevorsprünge zinnenförmig an dem Gabelzinken ausgebildet sein.

In einem weiteren Aspekt kann der Gabelträger stoffeinstückig mit den Gabelzinken ausgebildet sein (Integralbauweise). Alternativ können die Gabelzinken form- und/oder kraftschlüssig mit den Gabelzinken verbunden sein (Modularbauweise).

Die Aufgabe wird weiterhin gelöst durch ein System aus dem Handhabungswerkzeug, vorzugsweise nach einem der obigen Aspekte, und einem medizinischen Behälter, wobei der medizinische Behälter zumindest einen Falz beinhaltet und die Gabelzinken vorgesehen und ausgebildet sind, über die Aufnahmevorsprünge und/oder die Manipulationsvorsprünge den Falz des medizinischen Behälters formschlüssig zu kontaktieren und/oder zu fixieren.

In anderen Worten beinhaltet das System das Handhabungswerkzeug und den medizinischen Behälter, vorzugsweise in Form des Sterilgutcontainers. Die Aufnahmevorsprünge und/oder die Manipulationsvorsprünge sind in ihrer Geometrie auf eine Geometrie des Falzes des medizinischen Behälters abgestimmt. Anders ausgedrückt sind die Aufnahmevorsprünge und/oder die Manipulationsvorsprünge derart ausgebildet, dass sie in den Falz formschlüssig eingreifen.

In einem Aspekt kann der medizinische Behälter eine Grundfläche mit Abmessungen A x B aufweist und A ein Vielfaches von 1/3 einer Länge des Gabelzinkens in der Längsrichtung sein.

Anders ausgedrückt kann die Länge des Gabelzinkens auf die Abmessungen des medizinischen Behälters in einem definierten Verhältnis abgestimmt sein.

Durch eine derartige Ausbildung der Gabelzinken können eine Vielzahl von verschiedenen medizinischen Behältern bzw. verschieden große medizinische Behälter mittels des Handhabungswerkzeugs gehandhabt werden. Insbesondere können mit dem Handhabungswerkzeug Sterilgutcontainer mit einer Länge 1/1, 3/4 und 1/2 gehandhabt werden.

In einem Aspekt kann der Falz an gegenüberliegenden Seitenwänden des medizinischen Behälters symmetrisch ausgebildet sein.

In einem weiteren Aspekt kann der Falz parallel zu einem Boden und/oder einem Rand des medizinischen Behälters orientiert sein.

In einem weiteren Aspekt kann der Falz in einem einer Öffnung des medizinischen Behälters zugewandten Abschnitt des medizinischen Behälters ausgebildet sein. Anders ausgedrückt kann der Falz in einer Höhenrichtung in einem oberen Abschnitt des medizinischen Behälters ausgebildet sein.

In einem weiteren Aspekt kann der Falz einen Randabschnitt der Öffnung des medizinischen Behälters ausbilden.

### Kurzbeschreibung der Figuren

Fig. 1 ist eine Darstellung eines Handhabungswerkzeugs nach einem ersten Ausführungsbeispiel der vorliegenden Offenbarung;
Fig. 2 ist eine Darstellung eines Systems aus dem Handhabungswerkzeug nach dem ersten Ausführungsbeispiel und einem ersten Sterilgutcontainer;
Fig. 3 ist eine Schnittdarstellung des Schnitts A-A aus Fig. 2;
Fig. 4 ist eine Darstellung eines Systems aus dem Handhabungswerkzeug nach dem ersten Ausführungsbeispiel und einer halbformatigen Containerwanne in einer ersten Position;
Fig. 5 ist eine Darstellung des Systems aus dem Handhabungswerkzeug nach dem ersten Ausführungsbeispiel und der halbformatigen Containerwanne in einer zweiten Position;
Fig. 6 ist eine Darstellung des Handhabungswerkzeugs nach einem zweiten Ausführungsbeispiel der vorliegenden Offenbarung;
Fig. 7 ist eine Darstellung eines Systems aus dem Handhabungswerkzeug nach dem zweiten Ausführungsbeispiel und dem ersten Sterilgutcontainer;
Fig. 8 ist eine Schnittdarstellung des Schnittes B-B aus Fig. 7;
Fig. 9 ist eine Darstellung eines beispielhaften Regals mit einer Vielzahl an ersten Sterilgutcontainern;
Fig. 10 ist eine Darstellung eines Regalfachs des beispielhaften Regals und dem Handhabungswerkzeug nach dem zweiten Ausführungsbeispiel in einem ersten Handhabungsschritt; und
Fig. 11 ist eine Darstellung des Regalfachs des beispielhaften Regals und dem Handhabungswerkzeug nach dem zweiten Ausführungsbeispiel in einem zweiten Handhabungsschritt.

### Beschreibung der Ausführungsbeispiele

Nachstehend werden Ausführungsbeispiele der vorliegenden Offenbarung auf der Basis der zugehörigen Figuren beschrieben. Unter "oben bzw. Oberseite" wird nachfolgend ein höherer Wert in Z-Richtung/Höhenrichtung verstanden. Unter "unten bzw. Unterseite" wird nachfolgend ein geringerer Wert in Z-Richtung/Höhenrichtung verstanden. Unter "vorne" wird nachfolgend ein höherer Wert in X-Richtung/Längsrichtung verstanden. Unter "hinten" wird nachfolgend ein geringerer Wert in X-Richtung/Längsrichtung verstanden.

### Erstes Ausführungsbeispiel

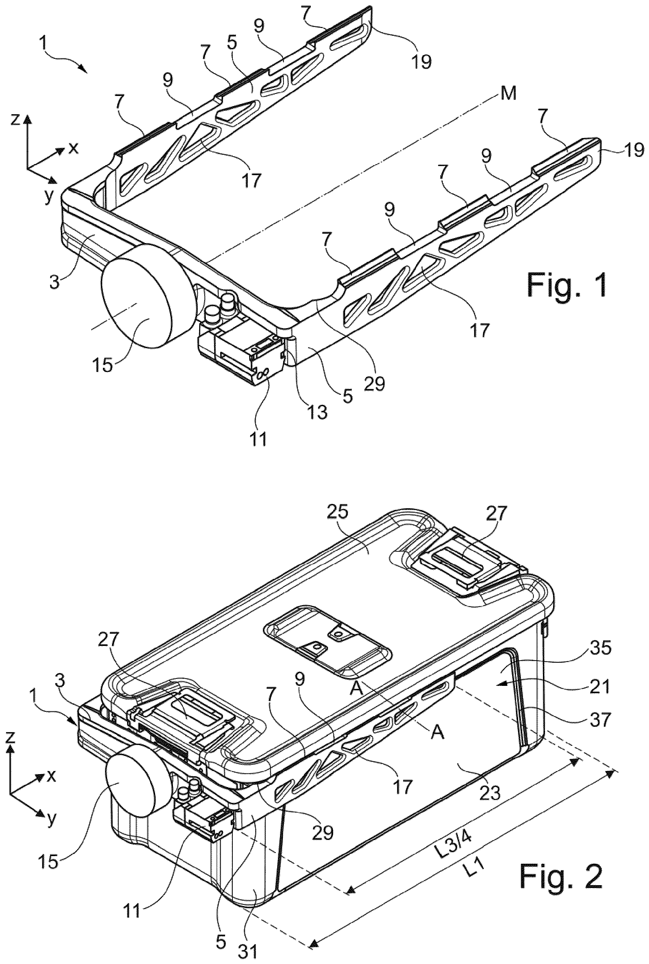

Fig. 1 zeigt eine perspektivische Ansicht eines offenbarungsgemäßen Handhabungswerkzeugs 1 in einem ersten Ausführungsbeispiel. Das Handhabungswerkzeug 1 beinhaltet einen Gabelträger 3, welcher in dem hier dargestellten ersten Ausführungsbeispiel als Doppel-T-Träger ausgebildet ist und sich in einer Breitenrichtung Y erstreckt. An Endabschnitten des Gabelträgers 3 ist jeweils ein Gabelzinken 5 ausgebildet, der sich rechtwinklig weg von dem Gabelträger 3 in einer Längsrichtung X erstreckt. Die zwei Gabelzinken 5 sind parallel ausgebildet. Der Gabelträger 3 und die zwei Gabelzinken 5 bilden im Wesentlichen eine U-Form. An einer Oberseite bzw. an einer Behälterauflage-/ erfassungsseite der Gabelzinken 5 sind Aufnahmevorsprünge 7 ausgebildet. Die Aufnahmevorsprünge 7 sind entlang der Längsrichtung X durch Aussparungen 9 unterbrochen. Anders ausgedrückt ist eine Erstreckung der Gabelzinken 5 in einer Höhenrichtung Z, welche normal zu einer von der Längsrichtung X und der Breitenrichtung Y aufgespannten Ebene orientiert ist, in Bereichen der Aufnahmevorsprünge 7 erhöht.

An dem Gabelträger 3 ist an einer von den Gabelzinken 5 abgewandten Seite ein Sensor 11 in einer Sensoraufnahme 13 ausgebildet. An der von den Gabelzinken 5 abgewandten Seite des Gabelträgers 3 ist weiterhin ein Interface 15 eines robotischen Systems (nicht dargestellt) ausgebildet. Das Interface 15 ist vorgesehen und ausgebildet, das Handhabungswerkzeug 1 im Raum zu bewegen und um eine Achse M zu rotieren. Die Gabelzinken 5 beinhalten Ausnehmungen 17, welche ausgebildet sind, ein Gewicht des Handhabungswerkzeugs 1 zu reduzieren. Durch die Ausnehmungen 17 weisen die Gabelzinken 5 eine belastungsgerechte Fachwerkgeometrie auf. Weiterhin beinhalten die Gabelzinken 5 an einem von dem Gabelträger 3 abgewandten Endabschnitt jeweils eine Fase 19.

In dem hier dargestellten ersten Ausführungsbeispiel sind der Gabelträger 3 und die zwei Gabelzinken 5 einstückig ausgebildet. Es sind auch Ausführungsformen vorstellbar, in denen die Gabelzinken 5 fest oder lösbar mit dem Gabelträger 3 verbunden sind.

Fig. 2 zeigt ein System aus dem offenbarungsgemäßen Handhabungswerkzeug 1 nach dem ersten Ausführungsbeispiel und einem medizinischen Behälter in Form eines Sterilgutcontainers 21. Der Sterilgutcontainer 21 beinhaltet eine Wanne 23 und einen Deckel 25. Die Wanne 23 und der Deckel 25 sind über den Verschluss 27 miteinander lösbar verbunden. Anders ausgedrückt verschließt der Deckel 25 die Wanne 23 des Sterilgutcontainers 21. Das Handhabungswerkzeug 1 ist vorgesehen und ausgebildet, den Sterilgutcontainer 21 zwischen den zwei Gabelzinken 5 aufzunehmen und den Sterilgutcontainer 21 automatisiert zu Greifen und handzuhaben.

Bei dem in Fig. 2 dargestellten Sterilgutcontainer 21 handelt es sich um einen vollformatigen bzw. einen 1/1 Sterilgutcontainer 21. Der vollformatige Sterilgutcontainer 21 weist in der Längsrichtung X eine Länge L1 auf. Die Gabelzinken 5 weisen in der Längsrichtung X eine Länge L3/4 auf. Die Länge L3/4 der Gabelzinken 5 entspricht dreiviertel der Länge L1 des vollformatigen Sterilgutcontainers 21.

An einem dem Gabelträger 3 zugewandten Endabschnitt des Gabelzinkens 5 ist ein Endanschlag 29 ausgebildet (vgl. auch Fig. 1). Bei dem Endanschlag 29 handelt es sich um eine teilsphärische/gekrümmte Ausnehmung, wobei ein Radius des Endanschlags 29 im Wesentlichen einem Eckradius einer Ecke 31 der Wanne 23 des Sterilgutcontainers 21 entspricht.

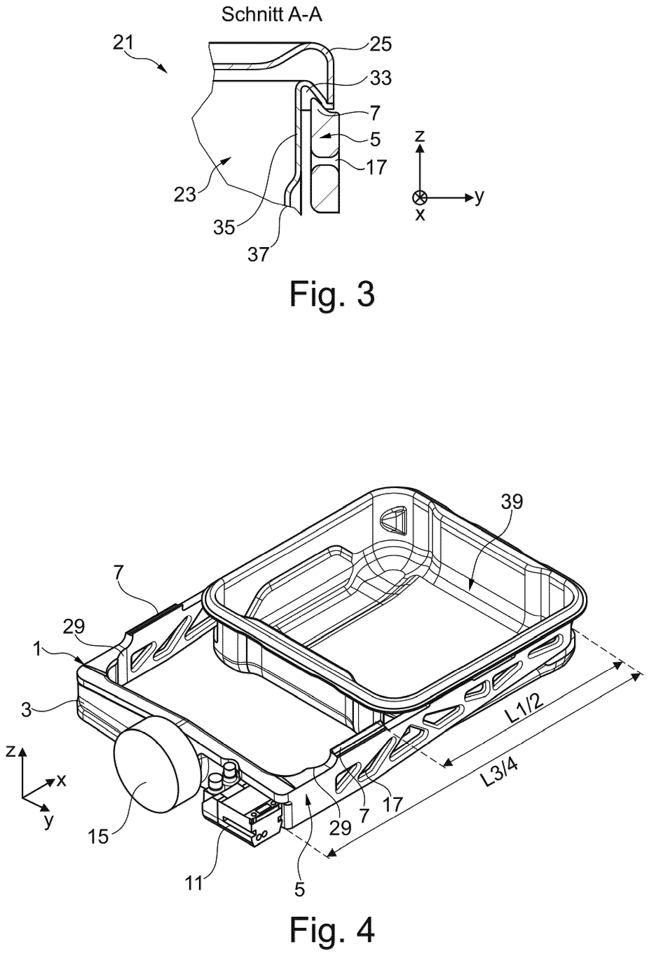

Das Handhabungswerkzeug 1 greift mit den Aufnahmevorsprüngen 7 in einen Falz 33 der Wanne 23 des Sterilgutcontainers 21 ein, was nachfolgend in Fig. 3 anhand des Schnittes A-A in Fig. 2 näher erläutert wird.

Fig. 3 zeigt den Schnitt A-A und damit einen Teilschnitt durch das System, welches in Fig. 2 dargestellt ist. Konkret zeigt Fig. 3 einen Schnitt durch den Gabelzinken 5 mit den Aufnahmevorsprüngen 7 und einen Kantenabschnitt des Sterilgutcontainers 21 mit der Wanne 23 und dem Deckel 25. Die Wanne 23 beinhaltet den Falz 33, der an einem Öffnungsrand der Wanne 23 zumindest an zwei gegenüberliegenden Seitenwänden 35 der Wanne 23 parallel ausgebildet ist. In dem hier dargestellten ersten Ausführungsbeispiel ist der Falz 33 stoffeinstückig mit der Seitenwand 35 ausgebildet und der Falz 33 und die Seitenwand 35 schließen einen spitzen Winkel ein.

Eine Wandstärke der Seitenwand 35 und der Falz 33 ist in dem hier dargestellten Ausführungsbeispiel konstant/identisch. Es sind jedoch Ausführungsbeispiele vorstellbar, in welchen die Wandstärke des Falz 33 größer ist als die Wandstärke der Seitenwand 35. Die Seitenwand 35 ist in dem hier dargestellten ersten Ausführungsbeispiel mit einer Versteifungssicke/Versteifungsstruktur 37 ausgebildet (vgl. auch Fig. 2).

Bei den Aufnahmevorsprüngen 7 handelt es sich um mit zunehmender Höhe in der Höhenrichtung Z bezogen auf eine Ausdehnung in der Breitenrichtung Y verschmälernde Geometrien. Anders ausgedrückt sind die Aufnahmevorsprünge 7 rampenförmige Geometrien, welche in Richtung hin zu der Achse M (vgl. Fig. 1) ansteigen. In nochmals anderen Worten ist zwischen den beiden Gabelzinken 5 eine Aufnahme für medizinische Behälter bzw. den Sterilgutcontainer 21 ausgebildet und die Höhe der Aufnahmevorsprünge 7 steigt in Höhenrichtung Z zu der Aufnahme hin an.

In dem hier dargestellten ersten Ausführungsbeispiel ist eine Steigung der Aufnahmevorsprünge 7 auf den Winkel der Falz 33 abgestimmt. In anderen Worten kontaktieren die Aufnahmevorsprünge 7 der Gabelzinken 5 die Falz 33 des Sterilgutcontainers 21, wenn der Sterilgutcontainer 21 sich in der Aufnahme des Handhabungswerkzeugs 1 befindet.

Nachfolgend wird eine Funktionsweise des Handhabungswerkzeugs 1 in Kombination mit dem vollformatigen Sterilgutcontainer 21 anhand von Fig. 2 und Fig. 3 beschrieben. Um den Sterilgutcontainer 21 handzuhaben und Pick- and-Place Aufgaben durchzuführen, wird das Handhabungswerkzeug 1 mittels eines robotischen Systems (nicht dargestellt), welches über das Interface 15 mit dem Handhabungswerkzeug 1 verbunden ist, in der Längsrichtung X verfahren, bis der Sterilgutcontainer 21 an dem Endanschlag 29 anliegt. Das Anliegen des Sterilgutcontainers 21 an dem Endanschlag 29 kann beispielsweise mittels des Sensors 11 überprüft/validiert werden.

Weiterhin kann der Sensor 11 ein an dem Sterilgutcontainer 21 angebrachtes Tag (nicht dargestellt) auslesen. Das Tag kann beispielsweise Informationen über einen Inhalt des Sterilgutcontainers 21 beinhalten. Weiterhin kann der Sensor 11 vorgesehen und ausgebildet sein, Tags, welche in/an einem Regal und/oder an einem Containertransportwagen angebracht sind und beispielsweise Informationen über einen Lagerort beinhalten, auszulesen.

Wenn der Sterilgutcontainer 21 sich an dem Endanschlag 29 befindet, wird das Verfahren des Handhabungswerkzeugs 1 in der Längsrichtung X gestoppt und das Handhabungswerkzeug 1 in positiver Höhenrichtung Z verfahren. Hierbei werden die Aufnahmevorsprünge 7 der Gabelzinken 5 in Eingriff/Formschluss mit dem Falz 33 des Sterilgutcontainers 21 gebracht und der Sterilgutcontainer 21 angehoben. In anderen Worten wird der Sterilgutcontainer 21 in einer senkrechten, translatorischen Bewegung des Handhabungswerkzeugs 1 in das Handhabungswerkzeug 1 eingesetzt, sodass der Sterilgutcontainer 21 mit dem Falz 33 in einem oberen Bereich zwischen den Gabelzinken 5 in der Aufnahme aufsitzt. Dies gewährleistet einen formschlüssigen definierten Sitz des zu transportierenden Sterilgutcontainers 21. Weiterhin stabilisiert der Sterilgutcontainer 21 selbst im eingesetzten Zustand die Gabelzinken 5 bezüglich einer seitlichen Bewegung.

Wenn der Sterilgutcontainer 21 derart formschlüssig über die Aufnahmevorsprünge 7 der Gabelzinken 5 mit dem Handhabungswerkzeug 1 verbunden ist, kann das Handhabungswerkzeug 1 mittels des robotischen Systems verfahren werden und der Sterilgutcontainer 21 umpositioniert werden. Um den Sterilgutcontainer 21 abzusetzen, verfährt das Handhabungswerkzeug 1 sobald der Sterilgutcontainer 21 auf einer Fläche aufsteht weiter in der negativen Höhenrichtung Z und die Aufnahmevorsprünge 7 und der Falz 33 werden außer Eingriff gebracht und damit der formschlüssige definierte Sitz gelöst.

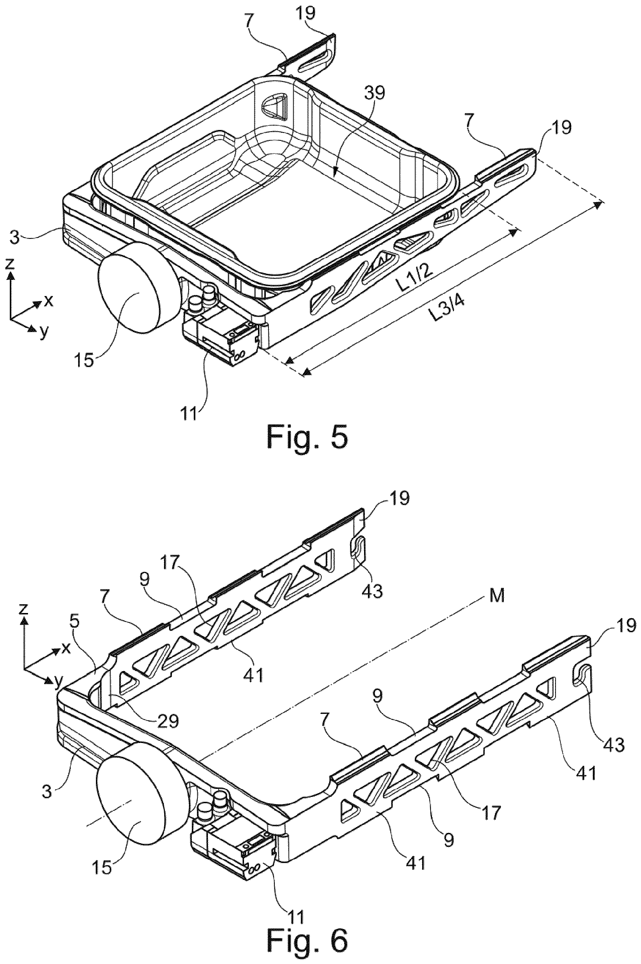

Fig. 4 und Fig. 5 zeigen das Handhabungswerkzeug 1 nach dem ersten Ausführungsbeispiel mit einem medizinischen Behälter in Form einer halbformatigen Containerwanne 39. Auf eine erneute Beschreibung des Handhabungswerkzeugs 1 nach dem ersten Ausführungsbeispiel wird verzichtet und es werden nachfolgend lediglich Unterschiede in einer Funktionsweise des Handhabungswerkzeugs 1 in Kombination mit der halbformatigen Containerwanne 39 anhand von Fig. 4 und Fig. 5 beschrieben.

Um die halbformatige Containerwanne 39 sicher handhaben zu können, ist es notwendig, die halbformatige Containerwanne 39 in Kontakt mit dem Endanschlag 29 zu bringen. Da bei der Handhabung der halbformatigen Containerwanne 39 die Länge L3/4 der Gabelzinken 5 länger ist als eine Länge L1/2 der halbformatigen Containerwanne 39, ist dies nicht ohne weiteres möglich, wenn die halbformatige Containerwanne 39 derart an einem Hindernis positioniert ist, dass die Gabelzinken 5 nicht so weit in der Längsrichtung X verfahren können, dass die halbformatige Containerwanne 39 mit dem Endanschlag 29 in Kontakt ist.

Fig. 4 zeigt einen ersten Schritt, in welchem die halbformatige Containerwanne 39 mit einem vorderen Abschnitt des Handhabungswerkzeugs 1 angehoben wird/ist. Die Eckabschnitte/Eckradien der halbformatigen Containerwanne 39 sind hierbei in den Aussparungen 9 angeordnet, um eine Kollision zwischen den Gabelzinken 5 und den Eckeradien der halbformatigen Containerwanne 39 zu verhindern und gleichzeitig einen definierten Anschlag in der Längsrichtung X für die halbformatige Containerwanne 39 in den Gabelzinken 5 auszubilden. In diesem ersten Schritt kann die halbformatige Containerwanne 39 mittels des Handhabungswerkzeugs 1 von dem Hindernis derart weg platziert werden, dass die Gabelzinken 5 in einem zweiten Schritt so in der Längsrichtung X verfahren können, bis die halbformatige Containerwanne 39 mit dem Endanschlag 29 in Kontakt ist, wie dies in Fig. 5 dargestellt ist.

In einem konkreten Beispiel kann somit die halbformatige Containerwanne 39, welche sich in einer zweiten Reihe in einem Regal und/oder Containertransportwagen befindet, mit dem vorderen Abschnitt/mit der Spitze des Handhabungswerkzeugs 1 angehoben werden und weiter nach vorn im Regal positioniert werden (erster Schritt). Anschließend kann die halbformatige Containerwanne 39 mit dem hinteren Teil des Handhabungswerkzeugs 1 erneut gehoben werden, wenn die Gabelzinken 5 über die halbformatige Containerwanne 39 hinausragen (zweiter Schritt). Dies hat den Vorteil, dass der medizinische Behälter unabhängig von seiner Größe mit einem möglichst kleinen Hebelarm zum Interface 15 des robotischen Systems verfahren werden kann und eine maximale Tragkraft des robotischen Systems möglichst gut ausgenutzt werden kann.

### Zweites Ausführungsbeispiel

Fig. 6 zeigt eine perspektivische Ansicht eines offenbarungsgemäßen Handhabungswerkzeugs 1 in einem zweiten Ausführungsbeispiel. Auf eine erneute Beschreibung von zu dem ersten Ausführungsbeispiel identischen Merkmalen und Eigenschaften wird nachfolgend verzichtet und es wird lediglich auf Unterschiede zu dem ersten Ausführungsbeispiel eingegangen.

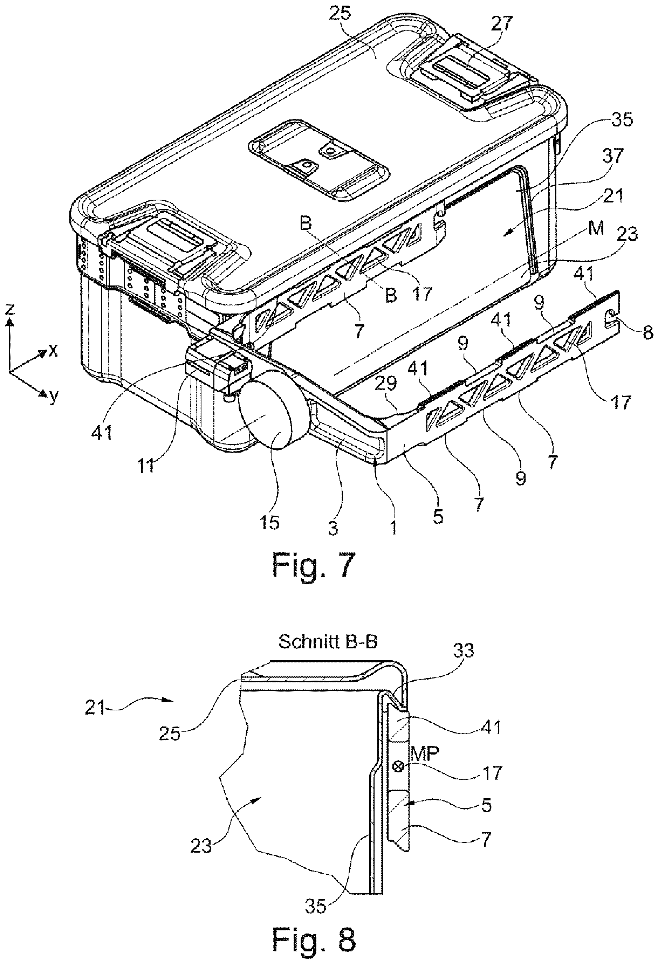

Das Handhabungswerkzeug 1 nach dem zweiten Ausführungsbeispiel beinhaltet an der Unterseite der Gabelzinken 5 Manipulationsvorsprünge 41. Als Unterseite wird die von der Oberseite abgewandte Seite des Gabelzinkens 5 verstanden. Anders ausgedrückt sind die Manipulationsvorsprünge 41 und die Aufnahmevorsprünge 7 an voneinander abgewandten Seiten des Gabelzinkens 5 ausgebildet. Die Manipulationsvorsprünge 41 sind entsprechend der Aufnahmevorsprünge 7 durch die Aussparungen 9 unterbrochen. Anders ausgedrückt sind die Aussparungen 9 an der Unterseite und an der Oberseite des Gabelzinkens 5 identisch angeordnet. Die genaue Geometrie der Manipulationsvorsprünge 41 wird später anhand von Fig. 8 näher erläutert.

An dem von dem Gabelträger 3 abgewandten Endabschnitt der Gabelzinken 5 sind weiterhin Rastelemente in Form von Haken 43 ausgebildet. Der Haken 43 ist ausgebildet, formschlüssig in an Objekten angebrachte Ösen oder Griffe oder dergleichen einzugreifen und das Objekt zu bewegen. In Fig. 6 ist das Handhabungswerkzeug 1 in einer Aufnahmestellung dargestellt. In der Aufnahmestellung zeigen die Aufnahmevorsprünge 7 nach oben bzw. in die positive Höhenrichtung Z.

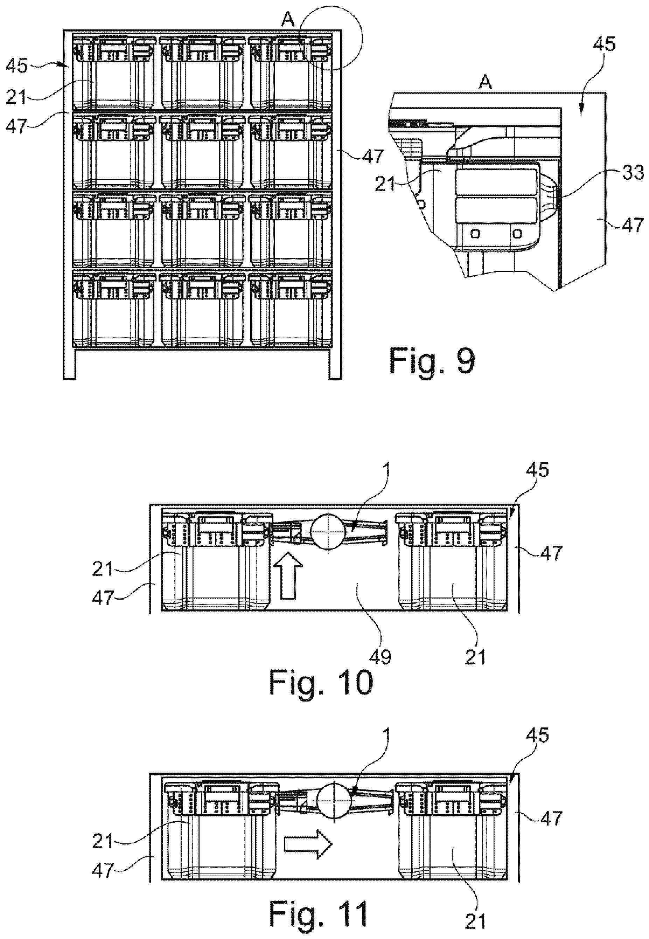

Fig. 7 zeigt das Handhabungswerkzeug 1 des zweiten Ausführungsbeispiels in einer Manipulationsstellung. Konkret ist das Handhabungswerkzeug 1 in der Manipulationsstellung um 180° um die Mittelachse M gedreht, sodass die Manipulationsvorsprünge 41 nun nach oben bzw. in die positive Höhenrichtung Z zeigen. In anderen Worten unterscheiden sich die Aufnahmestellung und die Manipulationsstellung in einer Drehung/Rotation des Handhabungswerkzeugs 1 um die Mittelachse um 180°. Die Drehung/Rotation des Handhabungswerkzeugs 1 erfolgt hierbei mittels des Interfaces 15 oder einer an das Interface 15 angeflanschten Rotationseinheit. Einer der Gabelzinken 5 ist mit den Manipulationsvorsprüngen 41 mit dem Falz 33 der Containerwanne 39 des Sterilgutcontainers 21 im Eingriff. Die genaue Funktionsweise wird später anhand von Fig. 9 bis Fig. 11 näher erläutert.

Fig. 8 zeigt einen Schnitt B-B aus Fig. 7. Der Manipulationsvorsprung 41 greift in den Falz 33 formschlüssig ein. In der hier dargestellten Manipulationsstellung ist der Aufnahmevorsprung 7 an einem von dem Falz 33 abgewandten Abschnitt des Gabelzinkens 5 ausgebildet. Der Manipulationsvorsprung 41 und der Aufnahmevorsprung 7 sind punktsymmetrisch bezüglich einer Gabelzinkenachse MP ausgebildet, welche sich in Längsrichtung X der Gabelzinke 5 erstreckt. Anders ausgedrückt entspricht die Kontur des Manipulationsvorsprungs 41 der Kontur des Aufnahmevorsprungs 7. In nochmals anderen Worten unterscheidet sich der Manipulationsvorsprung 41 von dem Aufnahmevorsprung 7 im Wesentlichen in seiner Orientierung an dem Gabelzinken 5.

Nachfolgend wird eine beispielhafte Funktionsweise des Handhabungswerkzeugs 1 des zweiten Ausführungsbeispiels anhand von Fig. 9 bis Fig. 11 beschrieben.

Fig. 9 zeigt ein beispielhaftes Regal 45, welches mit einer Vielzahl von Sterilgutcontainern (voll) besetzt ist. In der Vergrößerung A ist zu erkennen, dass der Falz 33 des Sterilgutcontainers 21 derart dicht an einer Wandung 47 des Regals 45 platziert ist, dass das Handhabungswerkzeug 1 nicht in den Falz 33 eingreifen kann.

In einem ersten Schritt (nicht dargestellt) wird ein mittig in dem Regal positionierter Sterilgutcontainer 21 aus der Anordnung in dem Regal 45 wie bereits oben beschrieben angehoben und gehandhabt. Nachdem ein Freiraum 49 durch ein Entfernen des mittleren Sterilgutcontainers 21 in dem Regal 45 entstanden ist, fährt das Handhabungswerkzeug 1 in einem zweiten Schritt in der Manipulationsstellung in den Freiraum 49 ein und die Manipulationsvorsprünge 41 werden mittels einer horizontalen und einer anschließenden vertikalen Bewegung in einem dritten Schritt in Eingriff mit dem Falz 33 des Sterilgutcontainers gebracht (siehe Fig. 10). Mit einer anschließenden weiteren horizontalen Bewegung wird der von dem Handhabungswerkzeug 1 eingehakte Sterilgutcontainer 21 in einem vierten Schritt von der Seitenwand 47 abgerückt/wegbewegt. Anschließend wird das Handhabungswerkzeug 1 in die Aufnahmestellung rotiert und der Sterilgutcontainer 21 (regulär) aufgenommen.

### Bezugszeichenliste

- 1: Handhabungswerkzeug
- 3: Gabelträger
- 5: Gabelzinken
- 7: Aufnahmevorsprung
- 9: Aussparung
- 11: Sensor
- 13: Sensoraufnahme
- 15: Interface
- 17: Ausnehmung
- 19: Fase
- 21: Sterilgutcontainer
- 23: Wanne
- 25: Deckel
- 27: Verschluss
- 29: Endanschlag
- 31: Ecke
- 33: Falz
- 35: Seitenwand
- 37: Versteifungssicke/Versteifungsstruktur
- 39: Containerwanne
- 41: Manipulationsvorsprung
- 43: Haken
- 45: Regal
- 47: Wandung
- 49: Freiraum
- X: Längsrichtung
- Y: Breitenrichtung
- Z: Höhenrichtung
- M: Achse
- MP: Gabelzinkenachse

## Patentansprüche

1. Handhabungswerkzeug (1), das zum Greifen und/oder Handhaben einer medizinischen Gerätschaft, vorzugsweise eines Behälters, weiter vorzugsweise Sterilisationsbehälters (21; 39), vorgesehen und ausgebildet ist, mit einem Gabelträger (3) und zwei sich in einer Längsrichtung (X) rechtwinklig weg vom Gabelträger (3) erstreckende, parallelbeabstandete Gabelzinken (5), von denen jeder eine Behälterauflage-/ erfassungsseite hat, an der eine Anzahl längsbeabstandeter Aufnahmevorsprünge (7) ausgebildet oder angeordnet sind, die sich in einer Höhenrichtung (Z) zur Behälterauflage-/ erfassungsseite erstrecken, **dadurch gekennzeichnet, dass** die Gabelzinken (5) eine von der Behälterauflage-/ erfassungsseite abgewandte Unterseite beinhalten, an welcher Manipulationsvorsprünge (41) ausgebildet oder angeordnet sind, die sich in einer Tiefenrichtung/ negativer Höhenrichtung (Z) zu der Unterseite erstrecken.

2. Handhabungswerkzeug (1) nach Anspruch 1, wobei die Aufnahmevorsprünge (7) der Gabelzinken (5) derart verjüngt, insbesondere keilförmig, ausgebildet sind, dass eine Höhenerstreckung der Aufnahmevorsprünge (7) in Höhenrichtung (Z) an zueinander zugewandten Seiten der Gabelzinken (5) größer ist als eine Höhenerstreckung der Aufnahmevorsprünge (7) in Höhenrichtung (Z) an voneinander abgewandten Seiten der Gabelzinken (5).

3. Handhabungswerkzeug (1) nach Anspruch 2, wobei die Manipulationsvorsprünge (41) derart verjüngt, insbesondere keilförmig, ausgebildet sind, dass eine Höhenerstreckung der Manipulationsvorsprünge (41) in Höhenrichtung (Z) an den zueinander zugewandten Seiten der Gabelzinken (5) kleiner ist als eine Höhenerstreckung der Manipulationsvorsprünge (41) in Höhenrichtung (Z) an den voneinander abgewandten Seiten der Gabelzinken (5).

4. Handhabungswerkzeug (1) nach Anspruch 3, wobei die Manipulationsvorsprünge (41) rotationssymmetrisch, vorzugsweise um 180° rotationssymmetrisch, zu den Aufnahmevorsprüngen (7), bezogen auf eine sich in Längsrichtung der Gabelzinken (5) erstreckenden Mittelachse (MP) der Gabelzinken (5), ausgebildet sind.

5. Handhabungswerkzeug (1) nach einem der Ansprüche 1 bis 4, wobei das Handhabungswerkzeug (1), vorzugsweise in / an dem Gabelträger (3), eine Sensoraufnahme (13) beinhaltet.

6. Handhabungswerkzeug (1) nach einem der Ansprüche 1 bis 5, wobei ein Abstand zwischen den zwei Gabelzinken (5), vorzugsweise stufenlos, veränderlich ist.

7. Handhabungswerkzeug (1) nach einem der Ansprüche 1 bis 6, wobei zumindest einer der Gabelzinken (5) an einem von dem Gabelträger (3) abgewandten Endabschnitt ein Rastelement, vorzugsweise einen Haken (43), beinhaltet.

8. Handhabungswerkzeug (1) nach einem der Ansprüche 1 bis 7, wobei zumindest einer der Gabelzinken (5) mit zumindest einem Klemmelement, vorzugsweise einem pneumatischen oder hydraulischen Kissen, ausgebildet ist.

9. Handhabungswerkzeug (1) nach einem der Ansprüche 1 bis 8, wobei die Gabelzinken (5) eine Fachwerkstruktur aufweisen.

10. Handhabungswerkzeug (1) nach einem der Ansprüche 1 bis 9, wobei der Gabelträger (3) an einem von den Gabelzinken (5) abgewandten Abschnitt ein Interface (15) eines robotischen Systems beinhaltet.

11. System aus dem Handhabungswerkzeug (1) nach einem der Ansprüche 1 bis 10, und einem medizinischen Behälter (21; 39), wobei der medizinische Behälter (21; 39) einen Falz (33) beinhaltet und die Gabelzinken (5) vorgesehen und ausgebildet sind, über die Aufnahmevorsprünge (7) den Falz (33) des medizinischen Behälters (21; 39) formschlüssig zu kontaktieren und/oder zu fixieren und/oder in den Falz (33) einzugreifen.

12. System nach Anspruch 11, wobei der medizinische Behälter (21; 39) eine Grundfläche mit Abmessungen A x B aufweist und A ein Vielfaches von 1/3 einer Länge des Gabelzinkens (5) in der Längsrichtung (X) ist.

## Claims

1. A handling tool (1), which is provided and configured for gripping and/or handling medical equipment, preferably a container, more preferably a sterilization container (21; 39), having a fork carriage (3) and two parallel-spaced fork tines (5) extending in a longitudinal direction (X) at right angles away from the fork carriage (3), each of which has a container support/capturing side on which a number of longitudinally spaced receiving protrusions (7) are formed or arranged, which extend in a height direction (Z) to the container support/capturing side, **characterized in that** the fork tines (5) include a lower side which faces away from the container support/capturing side and on which manipulation projections (41) are formed or arranged which extend in a depth direction/negative height direction (Z) to the lower side.

2. The handling tool (1) according to claim 1, wherein the receiving protrusions (7) of the fork tines (5) are tapered, in particular wedge-shaped, in such a way that a height extension of the receiving protrusions (7) in the height direction (Z) on sides of the fork tines (5) facing each other is greater than a height extension of the receiving protrusions (7) in the height direction (Z) on sides of the fork tines (5) facing away from each other.

3. The handling tool (1) according to claim 2, wherein the manipulation projections (41) are tapered, in particular wedge-shaped, in such a way that a height extension of the manipulation projections (41) in the height direction (Z) on the sides of the fork tines (5) facing each other is smaller than a height extension of the manipulation projections (41) in the height direction (Z) on the sides of the fork tines (5) facing away from each other.

4. The handling tool (1) according to claim 3, wherein the manipulation projections (41) are rotationally symmetrical, preferably rotationally symmetrical by 180°, to the receiving protrusions (7), relative to a center axis (MP) of the fork tines (5) extending in the longitudinal direction of the fork tines (5).

5. The handling tool (1) according to one of claims 1 to 4, wherein the handling tool (1) includes, preferably in/on the fork carriage (3), a sensor receptacle (13).

6. The handling tool (1) according to one of claims 1 to 5, wherein a distance between the two fork tines (5) is variable, preferably continuously variable.

7. The handling tool (1) according to one of claims 1 to 6, wherein at least one of the fork tines (5) includes a latching element, preferably a hook (43), at an end portion facing away from the fork carriage (3).

8. The handling tool (1) according to one of claims 1 to 7, wherein at least one of the fork tines (5) is formed with at least one clamping element, preferably a pneumatic or hydraulic cushion.

9. The handling tool (1) according to one of claims 1 to 8, wherein the fork tines (5) have a truss structure.

10. The handling tool (1) according to one of claims 1 to 9, wherein the fork carriage (3) includes an interface (15) of a robotic system on a portion facing away from the fork tines (5).

11. A system comprising the handling tool (1), preferably according to one of claims 1 to 10, and a medical container (21; 39), wherein the medical container (21; 39) contains a fold (33) and the fork tines (5) are provided and configured to contact and/or fix the fold (33) of the medical container (21; 39) in a form-fitting manner via the receiving protrusions (7) and/or to engage in the fold (33).

12. The system according to claim 11, wherein the medical container (21; 39) has a base area with dimensions A x B and A is a multiple of 1/3 of a length of the fork tines (5) in the longitudinal direction (X).

## Revendications

1. Outil de manutention (1), qui est prévu et conçu pour prendre et/ou manier un équipement médical, de préférence un récipient, de préférence un récipient de stérilisation (21; 39), avec un support de fourche (3) et deux dents de fourche (5) espacées parallèlement et s'étendant dans une direction longitudinale (X) à angle droit à l'écart du support de fourche (3), dont chacune présente un côté d'appui/de saisie de récipient sur lequel est formé ou disposé un certain nombre de saillies de réception (7) espacées longitudinalement qui s'étendent dans une direction de hauteur (Z) vers le côté d'appui/de saisie de récipient, **caractérisé en ce que** les dents de fourche (5) contiennent un côté inférieur tourné à l'opposé du côté d'appui/de saisie de récipient, sur lequel sont formées ou disposées des saillies de manipulation (41) qui s'étendent dans une direction de profondeur/direction de hauteur négative (Z) vers le côté inférieur.

2. Outil de manutention (1) selon la revendication 1, dans lequel les saillies de réception (7) des dents de fourche (5) sont conçues de manière effilée, en particulier en forme de coin, de sorte qu'une extension en hauteur des saillies de réception (7) dans la direction de hauteur (Z) sur des côtés des dents de fourche (5) tournés les uns vers les autres est plus grande qu'une extension en hauteur des saillies de réception (7) dans la direction de hauteur (Z) sur des côtés des dents de fourche (5) opposés les uns aux autres.

3. Outil de manutention (1) selon la revendication 2, dans lequel les saillies de manipulation (41) sont conçues de manière effilée, en particulier en forme de coin, de sorte qu'une extension en hauteur des saillies de manipulation (41) dans la direction de hauteur (Z) sur les côtés des dents de fourche (5) tournés les uns vers les autres est plus petite qu'une extension en hauteur des saillies de manipulation (41) dans la direction de hauteur (Z) sur les côtés des dents de fourche (5) opposés les uns aux autres.

4. Outil de manutention (1) selon la revendication 3, dans lequel les saillies de manipulation (41) sont conçues avec une symétrie de rotation, de préférence avec une symétrie de rotation de 180°, par rapport aux saillies de réception (7), par rapport à un axe central (MP) des dents de fourche (5) s'étendant dans la direction longitudinale des dents de fourche (5).

5. Outil de manutention (1) selon l'une quelconque des revendications 1 à 4, dans lequel l'outil de manutention (1) contient un logement de capteur (13), de préférence dans/sur le support de fourche (3).

6. Outil de manutention (1) selon l'une quelconque des revendications 1 à 5, dans lequel une distance entre les deux dents de fourche (5) est variable, de préférence en continu.

7. Outil de manutention (1) selon l'une quelconque des revendications 1 à 6, dans lequel au moins une des dents de fourche (5) contient un élément d'encliquetage, de préférence un crochet (43), sur une section d'extrémité opposée au support de fourche (3).

8. Outil de manutention (1) selon l'une quelconque des revendications 1 à 7, dans lequel au moins une des dents de fourche (5) est conçue avec au moins un élément de serrage, de préférence un coussin pneumatique ou hydraulique.

9. Outil de manutention (1) selon l'une quelconque des revendications 1 à 8, dans lequel les dents de fourche (5) présentent une ossature.

10. Outil de manutention (1) selon l'une quelconque des revendications 1 à 9, dans lequel le support de fourche (3) contient une interface (15) d'un système robotique sur une partie opposée aux dents de fourche (5).

11. Système composé de l'outil de manutention (1) selon l'une quelconque des revendications 1 à 10, et d'un récipient médical (21 ; 39), dans lequel le récipient médical (21 ; 39) contient un pli (33), et les dents de fourche (5) sont prévues et conçues pour entrer en contact et/ou fixer par complémentarité de formes le pli (33) du récipient médical (21 ; 39) par l'intermédiaire des saillies de réception (7) et/ou pour s'engager dans le pli (33).

12. Système selon la revendication 11, dans lequel le récipient médical (21 ; 39) présente une surface de base avec des dimensions A x B, et A est un multiple de 1/3 d'une longueur de la dent de fourche (5) dans la direction longitudinale (X).
